# EUROPÄISCHE PATENTSCHRIFT

(11) **EP 3 979 948 B1**
(45) Veröffentlichungstag und Bekanntmachung des Hinweises auf die Patenterteilung: **26.07.2023**
(21) Anmeldenummer: 20731049.1
(22) Anmeldetag: 04.06.2020
(51) Int. Cl.: A61F 2/16

(54) **INJEKTORANORDNUNG FÜR EIN EINFÜHREN EINER INTRAOKULARLINSE**
INJECTOR ASSEMBLY FOR INSERTING AN INTRAOCULAR LENS
AGENCEMENT D'INJECTEUR POUR L'INSERTION D'UNE LENTILLE INTRAOCULAIRE

(30) Priorität: 05.06.2019 DE 102019115125
(43) Veröffentlichungstag der Anmeldung: 13.04.2022
(73) Patentinhaber: Carl Zeiss Meditec AG, 07745 Jena (DE)
(72) Erfinder: KELP, Martin, 12205 Berlin (DE)
(74) Vertreter: PATERIS Patentanwälte PartmbB
(86) Internationale Anmeldenummer: PCT/EP2020/065512
(87) Internationale Veröffentlichungsnummer: WO 2020/245289

(56) Entgegenhaltungen:
- EP-A1- 0 302 278
- EP-A1- 0 830 083
- EP-B1- 0 830 083
- US-A1- 2015 342 726

## Beschreibung

Die Erfindung betrifft eine Injektoranordnung für ein Einführen einer Intraokularlinse in den Kapselsack eines Auges.

Bei der Kataraktbehandlung eines Auges wird herkömmlich nur ein kleiner Schnitt in die Hornhaut des Auges eingebracht, der so groß ist, dass eine Kanüle durch den Schnitt in das Auge eingeführt werden kann. Nachdem der Schnitt in die Hornhaut eingebracht wurde, wird die Linse des Auges mittels Phakoemulsifikation zerkleinert und anschließend aus dem Kapselsack eines Auges abgesaugt. Danach wird eine Intraokularlinse in das Auge eingesetzt. Dabei wird die Intraokularlinse gefaltet, so dass sie durch die Kanüle eines Injektors passt. Die Kanüle wird durch den Schnitt in den Kapselsack eingeführt und die gefaltete Intraokularlinse wird mittels des Injektors durch die Kanüle in den Kapselsack geschoben, in dem die Intraokularlinse sich entfaltet und somit die ursprüngliche Linse ersetzt.

Ein Kolben des Injektors kann von einem die Behandlung durchführenden Arzt von Hand gedrückt werden, oder der Arzt kann eine Antriebseinheit mit einem Motor verwenden, mittels der der Kolben verschoben wird. Nachteilig ist, dass herkömmliche Antriebseinheiten nur aufwändig oder unzureichend sterilisierbar sind. Dadurch können die Antriebseinheiten nur einmal verwendet werden und sind danach zu entsorgen.

US 2015/0342726 A1 offenbart eine Augenimplantationsvorrichtung mit einem Stößel, der nicht von Hand angetrieben ist. EP 0 302 278 A1 betrifft eine Injektionseinrichtung für die automatische Injektion von Pharmaka. WO 96/37152 A1 offenbart eine Linseninjektionsvorrichtung mit einem Stößel und Mittel zum Bereitstellen einer Kraft in einer Richtung entgegen der Vorschubrichtung des Stößels.

Aufgabe der Erfindung ist es daher, eine Injektoranordnung mit einem Injektor und einer Antriebseinheit zu schaffen, wobei die Antriebseinheit einfach sterilisierbar ist.

Die erfindungsgemäße Injektoranordnung weist einen Injektor, der einen Kolben und eine Kanüle aufweist und eingerichtet ist, eine Intraokularlinse mittels einer Translationsbewegung des Kolbens durch die Kanüle zu verschieben, eine Magnetkupplung und eine Antriebseinheit auf, die eine erste Kupplungshälfte der Magnetkupplung, einen Motor, der eingerichtet ist, die erste Kupplungshälfte in eine erste Rotationsbewegung anzutreiben, und ein Gehäuse aufweist, innerhalb dessen die erste Kupplungshälfte und der Motor eingekapselt sind und das einen ringförmigen Gehäuseabschnitt aufweist, der einen Kanal begrenzt, der einen kreisförmigen Querschnitt hat, wobei der Injektor eine zweite Kupplungshälfte der Magnetkupplung aufweist sowie der Injektor und die zweite Kupplungshälfte in dem Kanal angeordnet sind, wobei die erste Kupplungshälfte um den ringförmigen Gehäuseabschnitt herum angeordnet ist und somit eingerichtet ist, die erste Rotationsbewegung um den ringförmigen Gehäuseabschnitt durchzuführen und somit die zweite Kupplungshälfte mittels einem den ringförmigen Gehäuseabschnitt durchdringenden Magnetfeld der Magnetkupplung in eine zweite Rotationsbewegung anzutreiben, wobei der Injektor eingerichtet ist, die zweite Rotationsbewegung in die Translationsbewegung des Kolbens zu übersetzen.

Durch das Vorsehen der Magnetkupplung und dessen den ringförmigen Gehäuseabschnitt durchdringenden Magnetfeldes ist es möglich, das Gehäuse gekapselt auszuführen. Die Antriebseinheit mit dem gekapselten Gehäuse kann einfach sterilisiert werden, beispielsweise indem die Antriebseinheit in einem Autoklaven einer erhöhten Temperatur und einem erhöhten Druck ausgesetzt wird. Indem dadurch die Antriebeinheit einfach sterilisierbar ist, braucht die Antriebseinheit nicht nach jeder Behandlung entsorgt zu werden, sondern kann für weitere Kataraktbehandlungen wiederverwendet werden. Weil der Injektor in dem Kanal angeordnet ist, ist er vorteilhaft gegen ein radiales Verrutschen bezüglich der Längsachse des Injektors gesichert. Durch das Vorsehen der Antriebseinheit mit dem Motor ist es möglich, die zweite Kupplungshälfte mit einer besonders gleichmäßigen Geschwindigkeit zu drehen, wodurch ebenfalls die Translationsbewegung des Kolbens mit einer besonders gleichmäßigen Geschwindigkeit erfolgt. Dadurch bewegt sich die Intraokularlinse mit der besonders gleichförmigen Geschwindigkeit in der Kanüle, wodurch ein Haftgleiten der Intraokularlinse und eine damit möglicherweise einhergehende Beschädigung der Intraokularlinse wenig wahrscheinlich ist.

Es ist bevorzugt, dass die Antriebseinheit ein wiederverwendbares Bauteil ist und der Injektor ein Einwegbauteil aufweist. Dabei kann der gesamte Injektor ein Einwegbauteil sein. Alternativ ist denkbar, dass die zweite Kupplungshälfte ein wiederverwendbares Bauteil ist, das ebenfalls sterilisierbar ist, und der verbleibende Teil des Injektors ein Einwegbauteil ist.

Die erste Kupplungshälfte ist bevorzugt gleitend auf dem ringförmigen Gehäuseabschnitt gelagert. Dabei handelt es sich vorteilhaft um eine einfache und kostengünstige Form der Lagerung. Zudem erfüllt der ringförmige Gehäuseabschnitt eine Doppelfunktion, indem er zum einen einen Teil des Gehäuses bildet und damit dazu beiträgt, die erste Kupplungshälfte einzukapseln, und somit die Sterilisierung der Antriebseinheit erlaubt und zum anderen als Lager für die erste Kupplungshälfte wirkt. Um die erste Kupplungshälfte gleitend auf dem ringförmigen Gehäuseabschnitt zu lagern, kann die erste Kupplungshälfte einen nicht magnetischen Gleitring aufweisen, der eingerichtet ist, auf dem ringförmigen Gehäuseabschnitt zu gleiten. Alternativ zu dem nicht magnetischen Gleitring ist es denkbar, dass die erste Kupplungshälfte mehrere nicht magnetische Gleitringabschnitte aufweist, die in Umfangsrichtung voneinander beabstandet sind und eingerichtet sind, auf dem ringförmigen Gehäuseabschnitt zu gleiten.

Alternativ ist denkbar, dass ein nicht magnetisches Kugellager vorgesehen ist, mittels dessen die erste Kupplungshälfte auf dem ringförmigen Gehäuseabschnitt gelagert ist.

Es ist erfindungsgemäß, dass die erste Kupplungshälfte mindestens einen Permanentmagneten aufweist und die zweite Kupplungshälfte ein weichmagnetisches Material aufweist. Es ist bevorzugt, dass die zweite Kupplungshälfte aus dem weichmagnetischen Material besteht. Der Permanentmagnet magnetisiert das weichmagnetische Material und ist somit in der Lage, die zweite Kupplungshälfte in die zweite Rotationsbewegung anzutreiben. Das weichmagnetische Material ist weniger kostenintensiv als es der Fall wäre, wenn Permanentmagneten in der zweiten Kupplungshälfte vorgesehen werden würden.

Die erste Kupplungshälfte weist bevorzugt einen ringförmigen Permanentmagnethalter und mehrere Permanentmagneten auf, die an dem Permanentmagnethalter in einem Abstand zueinander in Umfangsrichtung des Permanentmagnethalters befestigt sind. Besonders bevorzugt ist hierbei, dass die Permanentmagneten gleichmäßig entlang des gesamten Umfangs des Permanentmagnethalters angeordnet sind.

Es ist bevorzugt, dass die zweite Kupplungshälfte einen Ring und mehrere von dem Ring radial bezüglich des Rings nach außen vorstehende Vorsprünge aufweist, wobei jeder der Vorsprünge genau einem der Permanentmagneten zugeordnet ist. Dadurch wird in jedem der Vorsprünge eine magnetische Polarisation erzeugt. Die magnetische Polarisierung in jedem der Vorsprünge führt dazu, dass die Kupplung zwischen der ersten Kupplungshälfte und der zweiten Kupplungshälfte relativ stark ist, wodurch eine relativ große Kraft von der ersten Kupplungshälfte auf die zweite Kupplungshälfte zum Antreiben der zweiten Kupplungshälfte übertragbar ist, ohne dass sich die zweite Kupplungshälfte an der ersten Kupplungshälfte unbeabsichtigt vorbei bewegt.

Bevorzugt sind die Permanentmagneten in Radialrichtung bezüglich des Permanentmagnethalters polarisiert. Dadurch werden die Vorsprünge mit einem besonders starken Magnetfeld beaufschlagt, wodurch die Kupplung zwischen der ersten Kupplungshälfte und der zweiten Kupplungshälfte besonders stark ist.

Die Permanentmagneten sind bevorzugt entlang des Umfangs des Rings jeweils abwechselnd zueinander entgegen gerichtet polarisiert. Dadurch verstärken sich die magnetischen Feldlinien von zwei benachbarten Permanentmagneten gegenseitig, wodurch die Kupplung zwischen der ersten Kupplungshälfte und der zweiten Kupplungshälfte ganz besonders stark ist.

Es ist bevorzugt, dass die Permanentmagneten jeweils eine radial bezüglich des Permanentmagnethalters nach innen gewandte konkave Oberfläche aufweisen, die eingerichtet ist, auf dem ringförmigen Gehäuseabschnitt zu gleiten, so dass die erste Kupplungshälfte gleitend auf dem ringförmigen Gehäuseabschnitt gelagert ist. Indem die Permanentmagneten und nicht etwa der Permanentmagnethalter auf dem ringförmigen Gehäuseabschnitt gleiten, ist der Reibungswiderstand bei der ersten Rotationsbewegung vergleichsweise gering. Es ist denkbar, dass die erste Kupplungshälfte eine nicht magnetische Beschichtung aufweist, die auf die konkaven Oberflächen aufgebracht ist. Dadurch wird erreicht, dass die Permanentmagneten nicht unmittelbar auf dem ringförmigen Gehäuseabschnitt gleiten. Alternativ ist denkbar, dass die Permanentmagneten gebildet sind von permanentmagnetischen Partikeln und einer Kunststoffmatrix, in die die permanentmagnetischen Partikel eingebracht sind.

Die Antriebseinheit weist bevorzugt ein Befestigungsmittel auf, das eingerichtet ist, den Injektor verdrehfest zu befestigen. Dadurch kann verhindert werden, dass sich der Injektor während der zweiten Rotationsbewegung dreht. Besonders bevorzugt befestigt das Befestigungsmittel den Injektor an einem Teil des Injektors, der verschieden von einem anderen Teil des Injektors ist, an dem die zweite Kupplungshälfte angreift.

Im Folgenden wird anhand der beigefügten schematischen Zeichnungen die Erfindung näher erläutert.

Figur 1 zeigt in einer schematischen Darstellung eine bevorzugte Ausführungsform der erfindungsgemäßen Injektoranordnung in verschiedenen Schnitten, wobei ein Injektor der Injektoranordnung zur Veranschaulichung außerhalb einer Antriebseinheit der Injektoranordnung angeordnet ist.

Figur 2 zeigt die Schnitte A-A und B-B aus Figur 1 der Injektoranordnung, wobei der Injektor an die Antriebseinheit montiert ist.

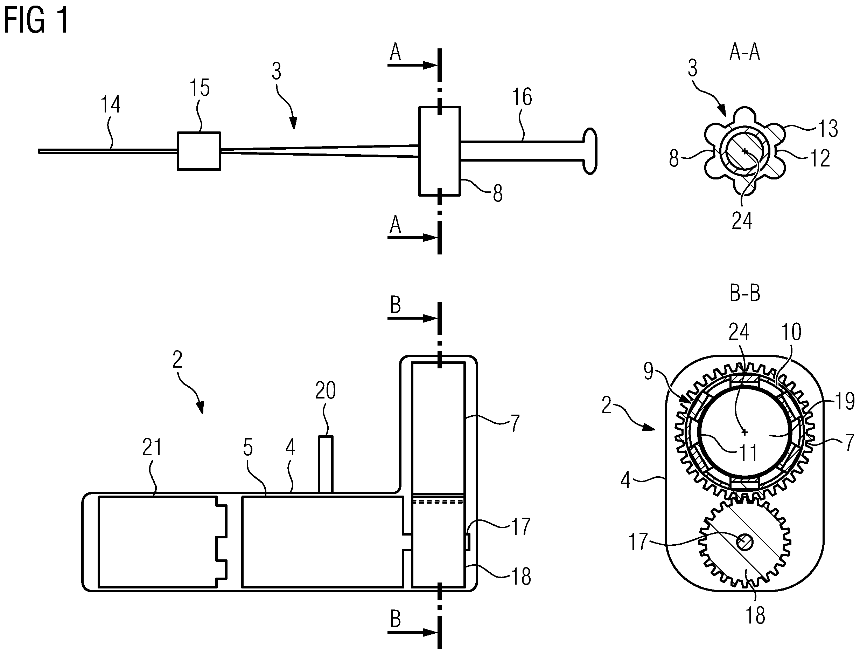

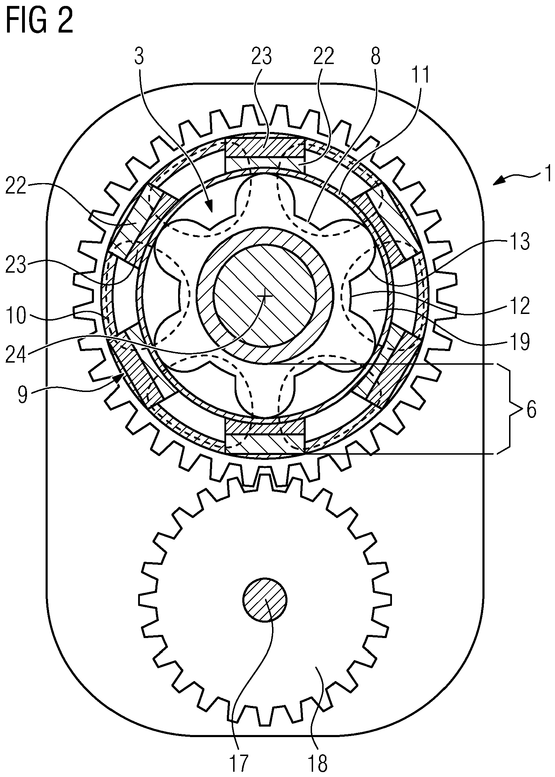

Wie es aus Figuren 1 und 2 ersichtlich ist, weist eine Injektoranordnung 1 einen Injektor 3, eine Antriebseinheit 2 und eine Magnetkupplung 6 auf. Der Injektor 3 weist eine Intraokularlinse, einen Kolben und eine Kanüle 14 auf und ist eingerichtet, die Intraokularlinse mittels einer Translationsbewegung des Kolbens durch die Kanüle 14 zu verschieben. Die Antriebseinheit 2 weist eine erste Kupplungshälfte 7 der Magnetkupplung 6, einen Motor 5 und ein Gehäuse 4 auf, innerhalb dessen die erste Kupplungshälfte 7 und der Motor 5 eingekapselt sind. Der Motor 5 ist eingerichtet, die erste Kupplungshälfte 7 in eine erste Rotationsbewegung anzutreiben. Das Gehäuse 4 weist einen ringförmigen Gehäuseabschnitt 11 auf, der einen Kanal 19 begrenzt, der einen kreisförmigen Querschnitt hat. Der Injektor 3 weist eine zweite Kupplungshälfte 8 der Magnetkupplung 6 auf, wobei der Injektor 3 und die zweite Kupplungshälfte 8 in dem Kanal 19 angeordnet sind. Die erste Kupplungshälfte 7 ist um den ringförmigen Gehäuseabschnitt 11 herum angeordnet und somit eingerichtet, die erste Rotationsbewegung um den ringförmigen Gehäuseabschnitt 11 durchzuführen und somit die zweite Kupplungshälfte 8 mittels eines den ringförmigen Gehäuseabschnitt 11 durchdringenden Magnetfeldes der Magnetkupplung 6 in eine zweite Rotationsbewegung anzutreiben. Der Injektor 3 ist eingerichtet, die zweite Rotationsbewegung in die Translationsbewegung des Kolbens zu übersetzen.

Die erste Rotationsbewegung und die zweite Rotationsbewegung können, wie in Figuren 1 und 2 dargestellt, um eine gemeinsame Rotationsachse 24 durchgeführt werden, wobei der Mittelpunkt des kreisförmigen Querschnitts des Kanals 19 auf der Rotationsachse 24 liegt.

Damit der Injektor 3 eingerichtet ist, die zweite Rotationsbewegung in die Translationsbewegung des Kolbens zu übersetzen, kann der Injektor 3 ein Schraubengetriebe aufweisen. Zum Bilden des Schraubengewindes kann der Injektor 3 beispielsweise, wie es die Figur 1 zeigt, einen Zylinder 16 aufweisen, innerhalb dessen der Kolben angeordnet ist. Der Zylinder 16 weist das Innengewinde auf und der Kolben weist das Außengewinde auf, wobei das Innengewinde und das Außengewinde miteinander in Eingriff stehen. Die zweite Kupplungshälfte 8 kann mit dem Kolben in Eingriff stehen, so dass die zweite Rotationsbewegung auch auf den Kolben übertragen wird, wodurch die Translationsbewegung des Kolbens erzeugt wird.

Das Gehäuse 4 kann, wie es auch Figur 1 zeigt, alle Bauteile der Antriebseinheit 2 vollständig einkapseln. In dem Fall, dass die Antriebseinheit 2 eine Stromversorgung 21, beispielsweise eine Batterie, aufweist, die eingerichtet ist, den Motor 5 mit Strom zu versorgen, ist auch die Stromversorgung 21 in dem Gehäuse 4 eingekapselt. Die Antriebseinheit 2 kann auch andere Bauteile aufweisen, wie zum Beispiel Sendeeinheiten und/oder Empfangseinheiten, die eingerichtet sind, den Motor 5 zu steuern. Die anderen Bauteile können ebenfalls von dem Gehäuse 4 eingekapselt sein.

Wie es aus Figur 1 ersichtlich ist, kann der Injektor 3 eine Kammer 15 aufweisen, in der die Intraokularlinse eingebracht ist. In diesem Fall ist der Kolben eingerichtet, die Intraokularlinse zuerst in die Kanüle 14 zu verschieben und anschließend aus dem der Kammer 15 abgewandten Ende der Kanüle 14 heraus zu verschieben. Die Kammer 14 kann eine Querschnittsverjüngung aufweisen, so dass bei der Translationsbewegung die Intraokularlinse gefaltet wird, bevor sie in die Kanüle 14 gelangt.

Es ist denkbar, dass die Antriebseinheit 2 ein wiederverwendbares Bauteil ist und der Injektor 3 ein Einwegbauteil aufweist. Dabei kann der gesamte Injektor 3 ein Einwegbauteil sein. Alternativ ist denkbar, dass die zweite Kupplungshälfte 8 ein wiederverwendbares Bauteil ist, das ebenfalls sterilisierbar ist, und der verbleibende Teil des Injektors 3 ein Einwegbauteil ist. In dem Fall, dass die zweite Kupplungshälfte 8 das wiederverwendbare Bauteil ist, kann eine kraftschlüssige und/oder formschlüssige Verbindung zwischen der zweiten Kupplungshälfte 8 und dem Kolben vorgesehen sein.

Figuren 1 und 2 zeigen, dass die erste Kupplungshälfte 7 einen ringförmigen Permanentmagnethalter 10 und mehrere Permanentmagneten 9 aufweist, die an dem Permanentmagnethalter 10 in einem Abstand zueinander in Umfangsrichtung des Permanentmagnethalters 10 befestigt sind. Die Permanentmagneten 9 sind an der Innenfläche des Permanentmagnethalters 10 befestigt und stehen somit von dem Permanentmagnethalter 10 radial bezüglich des Permanentmagnethalters 10 nach innen ab. Insbesondere ist eine gerade Anzahl der Permanentmagneten 9 vorgesehen, die gleichmäßig entlang des gesamten Umfangs des Permanentmagnethalters 10 verteilt sind. Die Permanentmagneten 9 sind in Radialrichtung bezüglich des Permanentmagnethalters 10 polarisiert, wobei die Permanentmagneten 9 entlang des Umfangs des Rings 12 jeweils abwechselnd zueinander entgegen gerichtet polarisiert sind. Dies wird dadurch erreicht, dass, wie in Figuren 1 und 2 dargestellt, bei einer ersten Gruppe der Permanentmagneten 9, die eine Hälfte der Permanentmagneten 9 aufweist, ein Nordpol 22 des Permanentmagneten 9 von dem Permanentmagnethalter 10 abgewandt angeordnet ist und ein Südpol 23 des Permanentmagneten 9 dem Permanentmagnethalter 10 zugewandt angeordnet ist. Bei einer zweiten Gruppe der Permanentmagneten 9, die die andere Hälfte der Permanentmagneten 9 aufweist, ist ein Südpol 23 des Permanentmagneten 9 dem Permanentmagnethalter 10 abgewandt angeordnet und ein Nordpol 22 des Permanentmagneten 9 dem Permanentmagnethalter 10 zugewandt angeordnet. Jeder der Permanentmagneten 9 aus der zweiten Gruppe ist dabei von zwei der Permanentmagneten 9 aus der ersten Gruppe benachbart.

Die zweite Kupplungshälfte 8 kann einen Ring 12 und mehrere von dem Ring 12 radial bezüglich des Rings 12 nach außen vorstehende Vorsprünge 13 aufweisen. Die Vorsprünge 13 sind gleichmäßig entlang des Umfangs des Rings 12 verteilt angeordnet und die Anzahl der Vorsprünge 13 ist gleich der Anzahl der Permanentmagneten 9, so dass jeder der Vorsprünge 13 genau einem der Permanentmagneten 9 zugeordnet ist. Die zweite Kupplungshälfte 8 kann ein weichmagnetisches Material aufweisen oder aus dem weichmagnetischen Material bestehen. Insbesondere können die Vorsprünge 13 und der Ring 12 ein weichmagnetisches Material aufweisen oder aus dem weichmagnetischen Material bestehen. Insbesondere weist die zweite Kupplungshälfte 8 keinen Permanentmagneten auf. Die gestrichelten Linien in Figur 2 stellen die Magnetfeldlinien des Magnetfeldes der Magnetkupplung 6 dar. Gut erkennbar ist, dass sich die Magnetfeldlinien von zwei benachbarten der Permanentmagneten 9 immer verstärken.

Wie es aus Figuren 1 und 2 ersichtlich ist, können die Permanentmagneten 9 jeweils eine radial bezüglich des Permanentmagnethalters 10 nach innen gewandte konkave Oberfläche aufweisen, die eingerichtet ist, auf dem ringförmigen Gehäuseabschnitt 11 zu gleiten, so dass die erste Kupplungshälfte 7 gleitend auf dem ringförmigen Gehäuseabschnitt 11 gelagert ist. Dabei kann der Krümmungsradius der konkaven Oberfläche identisch sein mit dem Krümmungsradius derjenigen Fläche des ringförmigen Gehäuseabschnitts 11, die in Kontakt mit der konkaven Oberfläche der Permanentmagneten 9 ist.

Figur 1 zeigt, dass die Antriebseinheit ein Befestigungsmittel 20 aufweisen kann, das eingerichtet ist, den Injektor 3 verdrehfest zu befestigen. Dabei kann das Befestigungsmittel 20 den Injektor 3 an einem Teil des Injektors 3 befestigen, der verschieden von dem Kolben ist. Bei dem Befestigungsmittel 20 kann es sich beispielsweise um eine Klammer handeln, die von dem Gehäuse 4 absteht.

Wie es aus Figur 1 ersichtlich ist, kann das Gehäuse 4 ein Gehäuseteil aufweisen, das von dem verbleibenden Gehäuse 4 absteht und den ringförmigen Gehäuseabschnitt 11 aufweist. Der Motor 5 ist in dem verbleibenden Teil des Gehäuses 4 angeordnet. In dem Fall, dass die Antriebseinheit 2 die Stromversorgung 21 aufweist, kann auch die Stromversorgung 21 in dem verbleibenden Teil des Gehäuses 4 angeordnet sein. Der Motor 5 weist eine von dem Motor 5 angetriebene Welle 17 auf und die Antriebseinheit 2 weist ein Antriebszahnrad 18 auf, das an der Welle 17 befestigt ist, zumindest teilweise in dem verbleibenden Teil des Gehäuses 4 angeordnet ist und von der Welle 17 angetrieben wird. Der Permanentmagnethalter 10 ist an seiner Außenseite als ein Zahnrad ausgebildet, das in das Antriebszahnrad 18 eingreift, so dass der Motor 5 eingerichtet, via die Welle 17, via das Antriebszahnrad 18 und die erste Kupplungshälfte 7 die zweite Kupplungshälfte 8 anzutreiben. In dem Fall, dass das Befestigungsmittel 20 vorgesehen ist, kann es in die gleiche Richtung wie das Gehäuseteil, das von dem verbleibenden Gehäuse 4 absteht, von dem Gehäuse 4 abstehen.

Die Antriebseinheit 2 kann einen Schalter aufweisen, der außen an dem Gehäuse 4 angebracht ist und eingerichtet ist, durch sein Betätigen den Motor 5 zu steuern. Alternativ ist denkbar, dass die Injektoranordnung einen Fernschalter aufweist, der eingerichtet ist, durch sein Betätigen den Motor 5 ferngesteuert zu steuern. Beispielsweise kann es sich bei dem Fernschalter um ein Fußpedal handeln.

### Bezugszeichenliste

1 Injektoranordnung
2 Antriebseinheit
3 Injektor
4 Gehäuse
5 Motor
6 Magnetkupplung
7 erste Kupplungshälfte
8 zweite Kupplungshälfte
9 Permanentmagnet
10 ringförmiger Permanentmagnethalter
11 ringförmiger Gehäuseabschnitt
12 Ring
13 Vorsprung
14 Kanüle
15 Kammer
16 Zylinder
17 Welle
18 Antriebszahnrad
19 Kanal
20 Befestigungsmittel
21 Stromversorgung
22 Nordpol
23 Südpol
24 Rotationsachse

## Patentansprüche

1. Injektoranordnung mit einem Injektor (3), der einen Kolben und eine Kanüle (14) aufweist und eingerichtet ist, eine Intraokularlinse mittels einer Translationsbewegung des Kolbens durch die Kanüle (14) zu verschieben, einer Magnetkupplung (6) und einer Antriebseinheit (2), die eine erste Kupplungshälfte (7) der Magnetkupplung (6), einen Motor (5), der eingerichtet ist, die erste Kupplungshälfte (7) in eine erste Rotationsbewegung anzutreiben, und ein Gehäuse (4) aufweist, innerhalb dessen die erste Kupplungshälfte (7) und der Motor (5) eingekapselt sind und das einen ringförmigen Gehäuseabschnitt (11) aufweist, der einen Kanal (19) begrenzt, der einen kreisförmigen Querschnitt hat, wobei der Injektor (3) eine zweite Kupplungshälfte (8) der Magnetkupplung (6) aufweist sowie der Injektor (3) und die zweite Kupplungshälfte (8) in dem Kanal (19) angeordnet sind, wobei die erste Kupplungshälfte (7) um den ringförmigen Gehäuseabschnitt (11) herum angeordnet ist und somit eingerichtet ist, die erste Rotationsbewegung um den ringförmigen Gehäuseabschnitt (11) durchzuführen und somit die zweite Kupplungshälfte (8) mittels einem den ringförmigen Gehäuseabschnitt (11) durchdringenden Magnetfeld der Magnetkupplung (6) in eine zweite Rotationsbewegung anzutreiben, wobei der Injektor (3) eingerichtet ist, die zweite Rotationsbewegung in die Translationsbewegung des Kolbens zu übersetzen, wobei die erste Kupplungshälfte (7) mindestens einen Permanentmagneten (9) aufweist und die zweite Kupplungshälfte (8) ein weichmagnetisches Material aufweist.

2. Injektoranordnung gemäß Anspruch 1, wobei die Antriebseinheit (2) ein wiederverwendbares Bauteil ist und der Injektor (3) ein Einwegbauteil aufweist.

3. Injektoranordnung gemäß Anspruch 1 oder 2, wobei die erste Kupplungshälfte (7) gleitend auf dem ringförmigen Gehäuseabschnitt (11) gelagert ist.

4. Injektoranordnung gemäß einem der Ansprüche 1 bis 3, wobei die erste Kupplungshälfte (7) einen ringförmigen Permanentmagnethalter (10) und mehrere Permanentmagneten (9) aufweist, die an dem Permanentmagnethalter (10) in einem Abstand zueinander in Umfangsrichtung des Permanentmagnethalters (10) befestigt sind.

5. Injektoranordnung gemäß Anspruch 4, wobei die zweite Kupplungshälfte (8) einen Ring (12) und mehrere von dem Ring (12) radial bezüglich des Rings (12) nach außen vorstehende Vorsprünge (13) aufweist, wobei jeder der Vorsprünge (13) genau einem der Permanentmagneten (9) zugeordnet ist.

6. Injektoranordnung gemäß Anspruch 4 oder 5, wobei die Permanentmagneten (9) in Radialrichtung bezüglich des Permanentmagnethalters (10) polarisiert sind.

7. Injektoranordnung gemäß Anspruch 6, wobei die Permanentmagneten (9) entlang des Umfangs des Rings (12) jeweils abwechselnd zueinander entgegen gerichtet polarisiert sind.

8. Injektoranordnung gemäß einem der Ansprüche 4 bis 7, wobei die Permanentmagnete (9) jeweils eine radial bezüglich des Permanentmagnethalters (10) nach innen gewandte konkave Oberfläche aufweisen, die eingerichtet ist, auf dem ringförmigen Gehäuseabschnitt (11) zu gleiten, so dass die erste Kupplungshälfte (7) gleitend auf dem ringförmigen Gehäuseabschnitt (11) gelagert ist.

9. Injektoranordnung gemäß einem der Ansprüche 1 bis 8, wobei die Antriebseinheit (2) ein Befestigungsmittel (20) aufweist, das eingerichtet ist, den Injektor (3) verdrehfest zu befestigen.

## Claims

1. Injector assembly with an injector (3), which has a plunger and a cannula (14) and is configured to move an intraocular lens through the cannula (14) by means of a translational movement of the plunger, a magnetic coupling (6) and a drive unit (2), which has a first coupling half (7) of the magnetic coupling (6), a motor (5), which is configured to drive the first coupling half (7) into a first rotational movement, and a housing (4), within which the first coupling half (7) and the motor (5) are encapsulated and which has an annular housing portion (11) which delimits a channel (19) which has a circular cross section, wherein the injector (3) has a second coupling half (8) of the magnetic coupling (6), and the injector (3) and the second coupling half (8) are arranged in the channel (19), wherein the first coupling half (7) is arranged around the annular housing portion (11) and is thus configured to carry out the first rotational movement around the annular housing portion (11) and therefore to drive the second coupling half (8) into a second rotational movement by means of a magnetic field of the magnetic coupling (6) penetrating the annular housing portion (11), wherein the injector (3) is configured to convert the second rotational movement into the translational movement of the plunger, wherein the first coupling half (7) has at least one permanent magnet (9) and the second coupling half (8) has a soft magnetic material.

2. Injector assembly according to Claim 1, wherein the drive unit (2) is a reusable component and the injector (3) has a disposable component.

3. Injector assembly according to Claim 1 or 2, wherein the first coupling half (7) is mounted in a sliding manner on the annular housing portion (11).

4. Injector assembly according to one of Claims 1 to 3, wherein the first coupling half (7) has an annular permanent magnet holder (10) and a plurality of permanent magnets (9) which are fastened to the permanent magnet holder (10) at a distance from one another in the circumferential direction of the permanent magnet holder (10) .

5. Injector assembly according to Claim 4, wherein the second coupling half (8) has a ring (12) and a plurality of projections (13) protruding outward from the ring (12) radially with respect to the ring (12), wherein each of the projections (13) is assigned to precisely one of the permanent magnets (9).

6. Injector assembly according to Claim 4 or 5, wherein the permanent magnets (9) are polarized in the radial direction with respect to the permanent magnet holder (10) .

7. Injector assembly according to Claim 6, wherein the permanent magnets (9) are polarized in each case in an alternating manner to one another in opposite directions along the circumference of the ring (12).

8. Injector assembly according to one of Claims 4 to 7, wherein the permanent magnets (9) each have a concave surface facing inward radially with respect to the permanent magnet holder (10), said surface being configured to slide on the annular housing portion (11), such that the first coupling half (7) is mounted in a sliding manner on the annular housing portion (11).

9. Injector assembly according to one of Claims 1 to 8, wherein the drive unit (2) has a fastening means (20) which is configured to fasten the injector (3) such that it does not rotate.

## Revendications

1. Agencement d'injecteur avec un injecteur (3), qui présente un piston et une canule (14) et qui est adapté pour déplacer une lentille intraoculaire au moyen d'un mouvement de translation du piston à travers la canule (14), un accouplement magnétique (6) et une unité d'entraînement (2) qui présente une première moitié d'accouplement (7) de l'accouplement magnétique (6), un moteur (5), qui est adapté pour entraîner la première moitié d'accouplement (7) dans un premier mouvement de rotation, et un boîtier (4) à l'intérieur duquel la première moitié d'accouplement (7) et le moteur (5) sont encapsulés et qui présente une section de boîtier annulaire (11) qui délimite un canal (19) ayant une section transversale circulaire, l'injecteur (3) présentant une deuxième moitié d'accouplement (8) de l'accouplement magnétique (6), et l'injecteur (3) et la deuxième moitié d'accouplement (8) étant agencés dans le canal (19), la première moitié d'accouplement (7) étant agencée autour de la section de boîtier annulaire (11) et étant ainsi adaptée pour effectuer le premier mouvement de rotation autour de la section de boîtier annulaire (11) et entraîner ainsi la deuxième moitié d'accouplement (8) dans un deuxième mouvement de rotation au moyen d'un champ magnétique de l'accouplement magnétique (6) pénétrant dans la section de boîtier annulaire (11), l'injecteur (3) étant adapté pour traduire le deuxième mouvement de rotation en le mouvement de translation du piston, la première moitié d'accouplement (7) présentant au moins un aimant permanent (9) et la deuxième moitié d'accouplement (8) présentant un matériau magnétique doux.

2. Agencement d'injecteur selon la revendication 1, dans lequel l'unité d'entraînement (2) est un composant réutilisable et l'injecteur (3) présente un composant à usage unique.

3. Agencement d'injecteur selon la revendication 1 ou 2, dans lequel la première moitié d'accouplement (7) est montée de manière coulissante sur la section de boîtier annulaire (11).

4. Agencement d'injecteur selon l'une quelconque des revendications 1 à 3, dans lequel la première moitié d'accouplement (7) présente un support annulaire d'aimants permanents (10) et plusieurs aimants permanents (9) fixés au support d'aimants permanents (10) à une distance les uns des autres dans la direction circonférentielle du support d'aimants permanents (10).

5. Agencement d'injecteur selon la revendication 4, dans lequel la deuxième moitié d'accouplement (8) présente une bague (12) et plusieurs saillies (13) faisant saillie radialement vers l'extérieur par rapport à la bague (12), chacune des saillies (13) étant associée à exactement un des aimants permanents (9).

6. Agencement d'injecteur selon la revendication 4 ou 5, dans lequel les aimants permanents (9) sont polarisés dans la direction radiale par rapport au support d'aimants permanents (10).

7. Agencement d'injecteur selon la revendication 6, dans lequel les aimants permanents (9) sont polarisés alternativement en sens inverse les uns des autres le long de la circonférence de la bague (12).

8. Agencement d'injecteur selon l'une quelconque des revendications 4 à 7, dans lequel les aimants permanents (9) présentent chacun une surface concave tournée radialement vers l'intérieur par rapport au support d'aimants permanents (10), qui est adaptée pour coulisser sur la section de boîtier annulaire (11), de telle sorte que la première moitié d'accouplement (7) est montée de manière coulissante sur la section de boîtier annulaire (11) .

9. Agencement d'injecteur selon l'une quelconque des revendications 1 à 8, dans lequel l'unité d'entraînement (2) présente un moyen de fixation (20) qui est adapté pour fixer l'injecteur (3) de manière immobile en rotation.
